# EUROPEAN PATENT SPECIFICATION

(11) **EP 4 340 868 B1**
(45) Date of publication and mention of the grant of the patent: **09.07.2025**
(21) Application number: 22724797.0
(22) Date of filing: 20.05.2022
(51) Int. Cl.: A61K 38/47, A61K 48/00, A61P 19/08

(54) **SECRETED SPLICING VARIANT OF KLOTHO FOR TREATING BONE DISORDERS**
SEKRETIERTE SPLEISSVARIANTE VON KLOTHO ZUR BEHANDLUNG VON KNOCHENERKRANKUNGEN
VARIANT D'ÉPISSAGE SÉCRÉTÉ DE KLOTHO POUR LE TRAITEMENT DE TROUBLES OSSEUX

(30) Priority: 21.05.2021 EP 21382465
(43) Date of publication of application: 27.03.2024
(73) Proprietor: Universitat Autònoma de Barcelona, 08193 Bellaterra (Cerdanyola del Vallès) (ES); Universitat de Barcelona, 08028 Barcelona (ES); Fundació Hospital Universitari Vall d'Hebron - Institut de Recerca, 08035 Barcelona (ES); Institució Catalana De Recerca I Estudis Avançats (ICREA), 08010 Barcelona (ES)
(72) Inventor: ROIG SORIANO, Joan, 12004 CASTELLÓ DE LA PLANA (ES); CHILLON RODRIGUEZ, Miguel, 08195 SANT CUGAT DEL VALLÈS (ES); BOSCH MERINO, Assumpció, 08195 SANT CUGAT DEL VALLÈS (ES); PALLÀS LLIBERIA, Mercè, 08028 BARCELONA (ES); GASPAR GRIÑAN, Christian, 08028 BARCELONA (ES)
(74) Representative: ZBM Patents - Zea, Barlocci & Markvardsen
(86) International application number: PCT/EP2022/063734
(87) International publication number: WO 2022/243519

(56) References cited:
- WO-A1-2004/100976
- WO-A1-2011/084452
- WO-A1-2017/085317
- WO-A1-2017/210607
- US-A1- 2017 233 446
- TOAN NGUYEN KHANH ET AL: "Soluble Klotho regulates bone differentiation by upregulating expression of the transcription factor EGRF-1", vol. 594, no. 2, 6 October 2019 (2019-10-06), NL, pages 290 - 300, XP055855643, ISSN: 0014-5793, Retrieved from the Internet <URL:https://onlinelibrary.wiley.com/doi/full-xml/10.1002/1873-3468.13613> DOI: 10.1002/1873-3468.13613
- SHALHOUB V ET AL: "Fibroblast Growth Factor 23 (FGF23) and Alpha-Klotho Stimulate Osteoblastic MC3T3.E1 Cell Proliferation and Inhibit Mineralization", CALCIFIED TISSUE INTERNATIONAL, SPRINGER-VERLAG, NE, vol. 89, no. 2, 3 June 2011 (2011-06-03), pages 140 - 150, XP019926456, ISSN: 1432-0827, DOI: 10.1007/S00223-011-9501-5

## Description

The present application claims the benefit of European Patent Application EP21382465 filed on 21 May 2021.

### Technical Field

The present invention relates to the field of medicine, in particular, it relates to medical approaches for preventing and/or treating bone disorders. The compounds of the invention are particularly useful for the treatment of age-related bone disorders.

### Background Art

Due to the increasing proportion of the aged population in modern societies, research in senescence hallmarks and methods to reverse them is a very active field of research. Senescence is understood as a gradual degenerative process that after adulthood leads to tissue dysfunction due to the deregulation of key cellular components such as epigenetic changes, mitochondrial function, intercellular communication, and metabolic pathways, among others. Those alterations are due to oxidative stress and inflammation, diet and other environmental factors, which generate accumulative damage and increase vulnerability to death.

Tissue degeneration is one of the main features associated with aging. Skeletal tissue is especially altered during this period, characterized by a progressive loss in bone mineral density (BMD), as well as alterations in the shape and structure of bone components. In particular, age has been showed to trigger bone geometric remodeling, tending to a medullary space and cortical perimeter expansion, due to endocortical bone absorption and periosteal bone apposition. This cortical bone expansion has been described in long bones of human and animal models for osteoporosis research and is explained as a compensatory mechanism to increase bone strength under bone loss, alterations in trabecular connectivity and low protein intake circumstances. All these age-related changes in skeletal tissue result in reduced bone quality, including a reduction in bone strength and flexibility, leading to an increased prevalence of fractures.

One of the main medicaments used for treating age-related bone degeneration is estrogen. However, some controversy exists over whether or not estrogen has any beneficial long-term effects. Furthermore, estrogen may carry the risk of increasing the prevalence of various types of tumors, such as breast and endometrial cancer. Calcitonin, osteocalcin with vitamin K, or high doses of dietary calcium, with or without vitamin D, have also been suggested for preventing bone loss in postmenopausal women. High doses of calcium, however, often have undesired gastrointestinal side effects, and serum and urinary calcium levels must be continuously monitored. Other current therapeutic approaches to osteoporosis include bisphosphonates, parathyroid hormone, calcilytics, calcimimetics, statins, anabolic steroids, lanthanum and strontium salts, and sodium fluoride. Such therapeutics, however, are often associated with undesirable side effects.

Klotho protein has been presented as an interesting candidate for treating all kind of age-related disorders due to its pleiotropic anti-aging protection and involvement in pathways that drive age-related chronic disorders such as kidney disease, tissue dysfunction, neurodegenerative diseases, and cancer, among others.

The Klotho gene is predominantly expressed in the kidney and choroid plexus in the brain and presents two main transcripts. Full-length mRNA transcribes a single-pass transmembrane protein called m-KL of 135KDa, composed of two extracellular domains (KL1 and KL2, of 72KDa and 60KDa respectively). The extracellular domains can be released from the membrane by protease-mediated shedding, generating soluble, circulating processed Klotho (p-KL, of 130KDa) which is sometimes simply called soluble Klotho. The alternative mRNA splicing shows a premature STOP codon and generates a secreted protein (s-KL, of 70KDa) containing just the KL1 domain and an extra 15 amino acids at its C-terminus. It must be noted that although a similar abbreviation (s-KL) is sometimes used in the prior art to name soluble Klotho (which is the processed version of transmembrane Klotho) and secreted Klotho (which is the splicing variant), these two variants present a different structure. In the present application the abbreviation s-KL is used to refer to secreted Klotho only.

Previous studies carried out in Klotho null mice (i.e. mice lacking the whole Klotho locus) have reported a positive effect of Klotho in maintaining bone structure (Maruyama, N., et al., "Bone micro-fragility caused by the mimetic aging processes in alpha-klotho deficient mice: in situ nanoindentation assessment of dilatational bands", Biomaterials, 2015. Vol. 47, pp. 62-71) and composition protection under normal and disease conditions, directly through FGF23 signaling and, indirectly by modulating the function of ion transporters and reducing cellular damage. In contrast, low KL levels generate an osteoporotic phenotype characterized by blood ion deregulation, low bone turnover and reduced fracture resistance (Kawaguchi, H., et al., "Independent impairment of osteoblast and osteoclast differentiation in klotho mouse exhibiting low-turnover osteopenia" J Clin Invest, 1999, vol. 104(3), p. 229-37; Suzuki, H., et al., "Histological and elemental analyses of impaired bone mineralization in klotho-deficient mice" J Anat, 2008. vol. 212(3), pp. 275-85).

However, the genetically modified mouse models used in the prior art do not allow to distinguish the relative effect of the different Klotho isoforms and, most importantly, they alter Klotho levels from the birth of the animals which may induce the apparition of compensatory effects that make it difficult to draw conclusions regarding the possible utility of Klotho-based treatments on adult mice.

In addition, it has been recently reported that exogenous Klotho administration causes bone hypomineralitzation in adult mice, which has hindered its use in the treatment of any bone condition (Minamizaki T. et al., "Soluble Klotho causes hypomineralization in Klotho-deficient mice" J Endocrinol., 2018, vol. 237(3), pp. 285-300).

Thus, in spite of the efforts made so far, there is still a need for efficient and safe treatments for bone disorders, in particular age-related bone disorders.

### Summary of Invention

The present inventors have developed a novel therapy for the prevention and/or treatment of bone disorders based on the administration of a particular splicing variant of Klotho.

As shown in the examples below, the present inventors have found that the administration of the secreted splicing variant of Klotho (s-KL) in a senescence-accelerated prone mouse model allows restoring bone parameters to values of a healthy mouse (see Table 1).

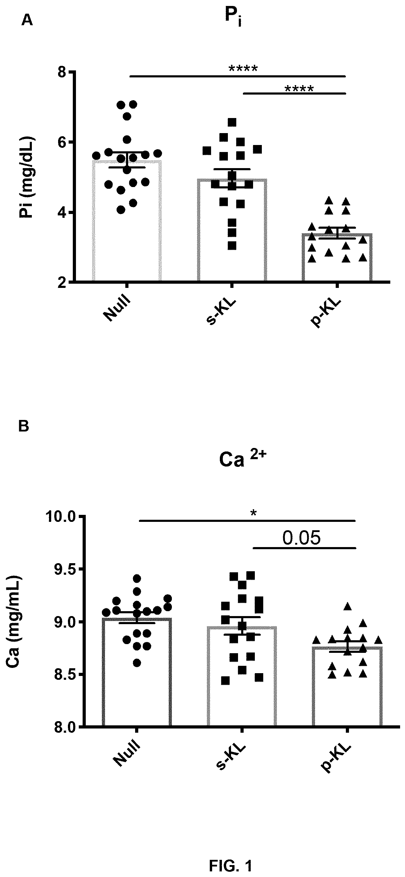

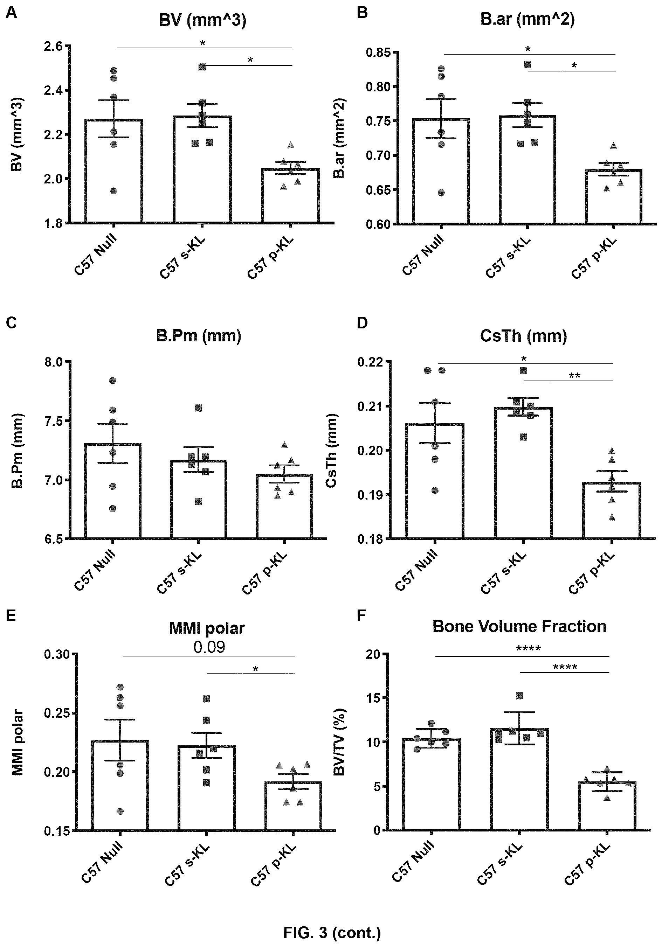

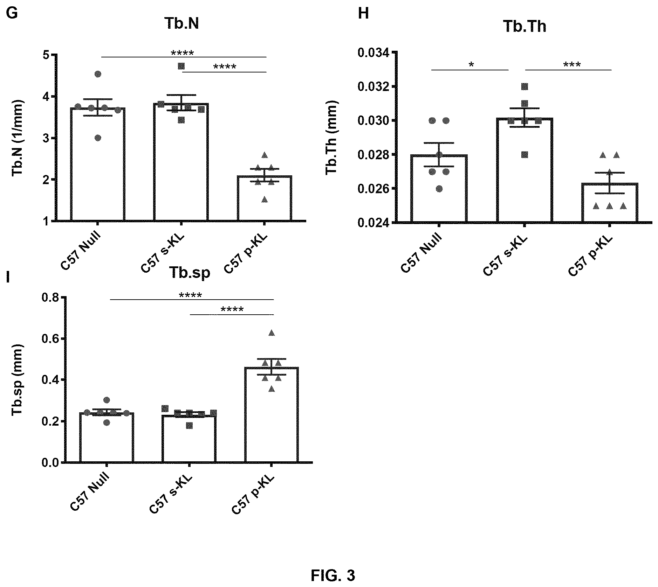

The role of s-KL herein reported is highly unexpected in light of the prior art as it was previously shown that the exogenous addition of processed Klotho (p-KL, sometimes also called soluble Klotho or s-KL) leads to bone toxicity in animals (Minamizaki T. et al., supra). However, the present inventors surprisingly found that secreted Klotho (s-KL-70KDa-, not to confuse with soluble Klotho -130KDa-) did not cause bone toxicity in contrast to p-KL (see Fig. 1, 2 and 3). Even more importantly, s-KL improved several bone parameters both in young animals and in senescence-accelerated mice, some of which even surpassed the values observed in control mice (see Figure 3 and Table 1).

Therefore, the results herein provided revealed that s-KL does not show the toxic side-effects in bone of the main Klotho variant and that it is even useful for the treatment of bone conditions.

Thus, in a first aspect, the present invention provides a polypeptide consisting of sequence SEQ ID NO: 1, or a variant thereof consisting of a sequence at least 85% identical to SEQ ID NO: 1, for use in the prevention and/or treatment of a bone disorder.

In a second aspect, the present invention provides a nucleic acid sequence that encodes the polypeptide or the variant thereof as defined in the first aspect, which is for use in the prevention and/or treatment of a bone disorder.

In a third aspect, the present invention provides a gene construct comprising a nucleic acid sequence as defined in the second aspect, operatively linked to an expression promoter, which is for use in the prevention and/or treatment of a bone disorder.

In a fourth aspect, the present invention provides and expression vector comprising the gene construct as defined in the third aspect, which is for use in the prevention and/or treatment of a bone disorder.

In a fifth aspect, the invention provides a host cell which is transformed or transfected with the nucleic acid sequence as defined in the second aspect, the gene construct as defined in the third aspect, or the expression vector as defined in the fourth aspect, which is for use in the prevention and/or treatment of a bone disorder.

### Brief Description of Drawings

Fig. 1 shows biochemical analysis of phosphate (A) and calcium (B) ions in serum of young animals treated with Null, s-KL or p-KL expressing AAVs.
Fig. 2 shows *fgf23* gene expression in bone tissue of young animals treated with Null, s-KL or p-KL expressing AAVs.
Fig. 3 shows MicroCT results of cortical and trabecular bone variables of young animals treated with Null, s-KL or p-KL expressing AAVs."BV" refers to bone volume; "B.ar" refers to bone area; "B.Pm" refers to bone perimeter; "MMI polar" refers to mean polar moment of inertia; "Tb.N" refers to trabecular number; "Tb.Th" refers to trabecular thickness and "Tb.sp" refers to trabecular space.
Fig. 4 shows the expression of genes involved in vitamin D activation and degradation (A and B) as well as the expression of ion channels genes in kidney (C and D) in young males (A and C) and females (B and D) treated with Null, s-KL or p-KL expressing AAVs. Data represented as fold change expression respect Null-treated animals.
Fig. 5 shows the effect of different KL isoforms over the expression of genes representative of different bone cell lineages in young males (A) and females (B) treated with Null, s-KL or p-KL expressing AAVs. Data represented as fold change expression respect Null-treated animals.
Fig. 6 shows the microCT mineral density analysis of cortical bone in young animals treated with Null, s-KL or p-KL expressing AAVs. The y axis represents BMD (Bone Mineral Density). (A) Males, (B) Females.
Fig. 7 shows the bone fracture test using 3 points bending test in young animals treated with Null, s-KL or p-KL expressing AAVs. (A) Males, (B) Females.
Fig. 8 shows KL isoforms (m-KL and s-KL) mRNA expression levels in liver (Lv), hippocampus (Hc) and cortex (Cx) tissue of treated animals. The y-axis represents fold change in the mRNA expression levels with respect of untreated animals.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

As used herein, the indefinite articles "a" and "an" are synonymous with "at least one" or "one or more." Unless indicated otherwise, definite articles used herein, such as "the" also include the plural of the noun.

As above discussed, in a first aspect, the invention provides a polypeptide consisting of sequence SEQ ID NO: 1, or a variant thereof consisting of a sequence at least 85% identical to SEQ ID NO: 1, for use in the prevention and/or treatment of a bone disorder.

This aspect can also be formulated as a polypeptide as defined above for use in the prevention and/or treatment of a bone disorder. The present invention also relates to a polypeptide as defined above for use in a method for the treatment and/or prevention of a bone disorder, comprising administering a therapeutically effective amount of a polypeptide as defined above, together with pharmaceutically acceptable excipients or carriers, in a subject in need thereof, including a human.

In a more particular embodiment of the first aspect of the invention, optionally in combination with any one of the embodiments provided below, the polypeptide consists of sequence SEQ ID NO: 1 or a variant thereof consisting of a sequence at least 85%, 86%, 87%, 88%, 88.5%, 89%, 89.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 99.5% identical to SEQ ID NO: 1. In an even more particular embodiment, the polypeptide consists of sequence SEQ ID NO: 1 or a variant thereof consisting of a sequence at least 88% or 98% identical to SEQ ID NO:1.

In another embodiment of the first aspect of the invention, optionally in combination with any one of the embodiments provided below, the polypeptide consists of sequence SEQ ID NO: 1 or SEQ ID NO: 2.

Protein variants are well understood to those of skill in the art and can involve amino acid sequence modifications. For example, amino acid sequence modifications typically fall into one or more of three classes: substitutional, insertional, or deletional variants.

In the present invention the term "identity" refers to the percentage of residues that are identical in the two sequences when the sequences are optimally aligned. If, in the optimal alignment, a position in a first sequence is occupied by the same amino acid residue as the corresponding position in the second sequence, the sequences exhibit identity with respect to that position. The percentage of identity determines the number of identical residues over a defined length in a given alignment. Thus, the level of identity between two sequences or ("percent sequence identity") is measured as a ratio of the number of identical positions shared by the sequences with respect to the number of positions compared (i.e., percent sequence identity = (number of identical positions/total number of positions compared) x 100). A gap, i.e., a position in an alignment where a residue is present in one sequence but not in the other, is regarded as a position with non-identical residues and is counted as a compared position.

As an illustration, by a polypeptide having an amino acid sequence having at least, for example, 95% identity to a reference amino acid sequence of SEQ ID NO:1 is intended that the amino acid sequence of the polypeptide is identical to the reference sequence except that the polypeptide sequence may include up to five amino acid alterations per each 100 amino acids of the reference amino acid of SEQ ID NO: 1. In other words, to obtain a polypeptide having an amino acid sequence of at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

A number of mathematical algorithms for rapidly obtaining the optimal alignment and calculating identity between two or more sequences are known and incorporated into a number of available software programs. For purposes of the present invention, the sequence identity between two amino acid sequences is preferably determined using algorithms based on global alignment, such as the Needleman-Wunsch algorithm (Needleman and Wunsch, 1970, J. Mol. Biol. 48: 443-453), preferably implemented in the Needle program of the EMBOSS package (EMBOSS: The European Molecular Biology Open Software Suite, Rice et al., 2000, Trends Genet. 16: 276-277); or the BLAST Global Alignment tool (Altschul et al., "Basic local alignment search tool", 1990, J. Mol. Biol, v. 215, pages 403-410), using default settings. Local alignment also can be used when the sequences being compared are substantially the same length.

The polypeptides with a percentage of identity of at least 88 % with any of SEQ ID NO: 1 or SEQ ID NO: 2 encompass s-KL of mammals other than mice and human.

SEQ ID NO: 1 is the amino acid sequence of the transcript from alternative splicing of α-klotho human gene, comprising the KL1 domain sequence, with an approximate weight of 70 kDa and a specific secretion signal consisting of 15 amino acid tail that is not found in the m-KL transcript. α-klotho human gene is the one located in Chromosome 13 NC_000013.11 (33016063..33066145) of the assembly GRCh38 (24.12.2013) for the human genome maintained by the Genome Reference Consortium. SEQ ID NO: 1 derives from the corresponding cDNA of SEQ ID NO: 3, deriving from the alternative splicing transcript of the mRNA sequence with the GenBank database accession number NM_004795 of 5012 base pairs, version 3 of 03.May.2014.

SEQ ID NO: 2 is the amino acid sequence of the transcript from alternative splicing of α-klotho mouse gene, comprising the KL1 domain sequence, with an approximate weight of 70 kDa with a specific secretion signal consisting of 15 amino acid tail that is not found in the m-KL transcript. α-klotho mouse gene is the one located in Chromosome 5 (150,952,607-150,993,809) of UCSC Genome Browser on Mouse July 2007 (NCBI37/mm9) Assembly for the mouse genome. SEQ ID NO: 2 derived from the corresponding cDNA of SEQ ID NO: 4, deriving in turn from the alternative splicing transcript of the mRNA sequence with the GenBank database accession number NM_013823 of 5124 base pairs, version 2 of 15.February.2015.

In another embodiment of the first aspect of the invention, optionally in combination with any one of the embodiments provided below, the polypeptide variant consists of sequence SEQ ID NO: 5 or SED ID NO: 6.

In another embodiment of the first aspect of the invention, optionally in combination with any one of the embodiments provided below, the polypeptide has a length equal to or lower than 645 amino acids, 600 amino acids, or 550 amino acids. In an even more particular embodiment, the polypeptide consists of sequence SEQ ID NO: 1 or a variant thereof consisting of a sequence at least 85% identical to SEQ ID NO: 1 and has a length equal to or lower than 645 amino acids, 600 amino acids, or 550 amino acids. In a particular embodiment, the polypeptide consists of sequence SEQ ID NO: 1 or a variant thereof consisting of a sequence at least 85% identical to SEQ ID NO: 1, and said variant has a length selected from the group consisting of 545, 546, 547, 548, 549, 550, 551, 552, 553, 554, 555, 556, 557, 558, 559, 560, 561, 562, 563, 564, 565, 566, 567, 568, 569, 570, 571, 572, 573, 574, 575, 576, 577, 578, 579, 580, 581, 582, 583, 584, 584, 586, 587, 588, 589, 590, 591, 592, 593, 594, 595, 596, 597, 598, 599, and 600 amino acids, or a length from 545 to 600 amino acids.

In one embodiment of the first aspect of the invention, optionally in combination with any one of the embodiments provided below, the polypeptide is the secreted splicing variant of mammalian klotho protein (s-KL). In an even more particular embodiment, the polypeptide is the human s-KL. The secreted splicing variant of mammalian Klotho protein (s-KL) has been disclosed in the prior art (see, for example, WO2017085317A1). Thus, the invention can be in particular formulated as secreted splicing variant of mammalian Klotho protein (s-KL), in particular human s-KL, or nucleic acid sequence coding therefor, for use in the prevention and/or treatment of a bone disorder.

The term "secreted splicing variant of mammalian Klotho" abbreviated as "s-KL", refers to the protein resulting from the transcript from alternative splicing, which generates a truncated form of the protein (s-KL) that is formed by the KL1 domain, with an approximate weight of 70 kDa, together with a specific secretion signal consisting of 15 amino acid tail that is not found in the m-KL transcript, and for this reason is also called the secreted isoform of klotho, s-KL, or the secreted splicing variant of klotho protein. s-KL is different from other forms of soluble klotho, namely p-KL, p-KL1 and p-KL2. In this description, m-KL stands for the full-length transmembrane form; p-KL stands for the soluble proteolyzed klotho, which is generated by cleavage of the m-KL; and p-KL1 and p-KL2 stand for the soluble klotho forms consisting on the KL1 domain and the KL2 domain of p-KL, respectively. m-KL comes from the full-length transcript encoding a single pass transmembrane protein with a molecular weight of approximately 130 kDa (m-KL). The protein contains three domains: a short transmembrane domain at the C-terminal, an extracellular domain composed of two internal repeated sequences of about 550 amino acids called KL1 and KL2 respectively, and a very short intracellular domain of 10 amino acids. The extracellular domain of the transmembrane form can be cleaved by metalloproteinases ADAM10 and ADAM17 resulting in another form of soluble Klotho of about 130 kDa (abbreviated p-KL for proteolyzed membrane isoform. Moreover, there is a second recognition site for the proteases ADAM10 and 17 located between the KL1 and KL2 domains, which generates two new 70 kDa isoforms, one contained the KL1 domain only (like the one generated from alternative splicing but without the specific amino acid tail), and the other one contained the KL2 domain. However, it has not been demonstrated *in vivo* that p-KL is proteolyzed into p-KL1 and p-KL2.

As used herein, "bone disorder" refers to a disease or condition characterized by a decrease in bone mass and/or an alteration in bone structure, leading to a decrease in bone strength and elasticity and a higher probability of fractures. Bone strength and elasticity may be measured by micro-computed tomography (micro-CT) or by performing a bending test according to standard protocols.

In one embodiment of the first aspect of the invention, optionally in combination with any one of the embodiments provided below, the bone disorder is bone degeneration and/or bone loss.

As used herein, "bone degeneration" refers to any condition where the structural integrity of the skeletal mass is altered. As used herein, the term "bone loss" refers to a situation in which skeletal mass is decreased, for instance by a reduction in bone density.

In one embodiment of the first aspect of the invention, optionally in combination with any one of the embodiments provided below, the bone disorder is age-related bone degeneration and/or bone loss.

In another embodiment of the first aspect of the invention, optionally in combination with any one of the embodiments provided below, the bone disorder is osteopenia and/or osteoporosis.

In another embodiment of the first aspect of the invention, optionally in combination with any one of the embodiments provided below, the bone disorder is caused by achondroplasia, cleidocranial dysostosis, enchondromatosis, fibrous dysplasia, Gaucher's Disease, hypophosphatemic rickets, Marfan's syndrome, multiple hereditary exotoses, neurofibromatosis, osteogenesis imperfecta, osteopoikilosis, sclerotic lesions, pseudoarthrosis, pyogenic osteomyelitis, periodontal disease, anti-epileptic drug induced bone loss, primary and secondary hyperparathyroidism, familial hyperparathyroidism syndromes, weightlessness induced bone loss, osteoporosis in men, postmenopausal bone loss, osteoarthritis, renal osteodystrophy, infiltrative disorders of bone, oral bone loss, osteonecrosis of the jaw, juvenile Paget's disease, melorheostosis, metabolic bone diseases, mastocytosis, sickle cell anemia/disease, organ transplant related bone loss, kidney transplant related bone loss, systemic lupus erythematosus, ankylosing spondylitis, epilepsy, juvenile arthritides, thalassemia, mucopolysaccharidoses, fabry disease, turner syndrome, Down Syndrome, Klinefelter Syndrome, leprosy, Perthes' Disease, adolescent idiopathic scoliosis, infantile onset multi-system inflammatory disease, Winchester Syndrome, Menkes Disease, Wilson's Disease, ischemic bone disease (such as Legg-Calve-Perthes disease, regional migratory osteoporosis), anemic states, conditions caused by steroids, glucocorticoid-induced bone loss, heparin-induced bone loss, bone marrow disorders, scurvy, malnutrition, calcium deficiency, idiopathic osteopenia or osteoporosis, congenital osteopenia or osteoporosis, alcoholism, chronic liver disease, postmenopausal state, chronic inflammatory conditions, rheumatoid arthritis, inflammatory bowel disease, ulcerative colitis, inflammatory colitis, Crohn's disease, oligomenorrhea, amenorrhea, pregnancy, diabetes mellitus, hyperthyroidism, thyroid disorders, parathyroid disorders, Cushing's disease, acromegaly, hypogonadism, immobilization or disuse, reflex sympathetic dystrophy syndrome, regional osteoporosis, osteomalacia, bone loss associated with joint replacement, HIV-associated bone loss, bone loss associated with loss of growth hormone, bone loss associated with cystic fibrosis, fibrous dysplasia, chemotherapy-associated bone loss, tumor induced bone loss, cancer-related bone loss, hormone-ablative bone loss, multiple myeloma, drug-induced bone loss, anorexia nervosa, disease associated facial bone loss, disease associated cranial bone loss, disease associated bone loss of the jaw, disease associated bone loss of the skull, or bone loss associated with space travel.

In a particular embodiment of the first aspect, optionally in combination with any of the embodiments provided above and below, the polypeptide is for use in preventing, treating, and/or reducing bone loss; preventing, treating, and/or reducing bone fracture; preventing, treating and/or reducing bone fragility; increasing bone mass; increasing bone structural integrity; increasing trabecular thickness; increasing bone mineral density; or combinations thereof.

In a particular embodiment of the first aspect, optionally in combination with any of the embodiments provided above and below, the polypeptide is linked to a heterologous moiety.

As used herein, "heterologous moiety" refers to any molecule coupled to the polypeptide via either a covalent or non-covalent bond. In a particular embodiment, the heterologous moiety is located in either the N-terminal or the C-terminal end of the polypeptide. In a particular embodiment, the heterologous moiety is located in both the N-terminal and the C-terminal ends of the polypeptide.

The heterologous moiety can be, for example, a molecule that facilitates the purification of the polypeptide. In a particular embodiment, the heterologous moiety is a peptide. In an even more particular embodiment, the heterologous moiety is a poly histidine track. As the skill in the art would understand, small peptides that assist in the purification of the protein can be maintained in the final compound without affecting its functionality.

The heterologous moiety can also be any vehiculization agent to facilitate the absorption, transport and delivery of the polypeptide.

These polypeptides resulting from KL protein, such as in particular s-KL, may be used directly in the form of the protein, or they (e.g., s-KL) can be expressed inside target cells of the tissue of interest by means of gene therapy. To this aim the invention also provides, in a second aspect, a nucleic acid sequence that encodes the polypeptide or the variant thereof as defined in the first aspect, which is for use in the prevention and/or treatment of a bone disorder.

The term "a nucleic acid sequence that encodes the polypeptide" is to be understood, in particular, as the cDNA sequence resulting from the reverse transcription (RT-PCR) of the mRNA coding for said polypeptide.

This aspect can also be formulated as the use of the nucleic acid sequence as defined above for use in the prevention and/or treatment of a bone disorder. The present invention also relates to the nucleic acid sequence as defined above for use in a method for the treatment and/or prevention of a bone disorder, comprising administering a therapeutically effective amount of the nucleic acid sequence as defined above, together with pharmaceutically acceptable excipients or carriers, in a subject in need thereof, including a human.

In a particular embodiment of the second aspect, optionally in combination with any of the embodiments provided above and below, the nucleic acid sequence comprises SEQ ID NO: 3 or SEQ ID NO: 4. In an even more particular embodiment, the nucleic acid sequence consists of SEQ ID NO: 3 or SEQ ID NO: 4.

In a more particular embodiment of the second aspect of the invention, optionally in combination with any one of the embodiments provided below, the nucleic acid sequence consists of sequence SEQ ID NO: 3 or SEQ ID NO: 4 or a variant thereof consisting of a sequence at least 85%, 86%, 87%, 88%, 88.5%, 89%, 89.5%, 90%, 90.5%, 91%, 91.5%, 92%, 92.5%, 93%, 93.5%, 94%, 94.5%, 95%, 95.5%, 96%, 96.5%, 97%, 97.5%, 98%, 98.5%, 99%, or 99.5% identical to SEQ ID NO: 3 or SEQ ID NO: 4.

In a third aspect, the invention provides a gene construct comprising a nucleic acid sequence as defined in the second aspect operatively linked to an expression promoter, which is for use in the prevention and/or treatment of a bone disorder.

This aspect can also be formulated as a gene construct as defined above for use in the prevention and/or treatment of a bone disorder. The present invention also relates to the gene construct as defined above for use in a method for the treatment and/or prevention of a bone disorder, comprising administering a therapeutically effective amount of a gene construct as defined above, together with pharmaceutically acceptable excipients or carriers, in a subject in need thereof, including a human.

In a particular embodiment of the third aspect, optionally in combination with any of the embodiments provided above and below, the expression promoter operatively linked is selected from the group consisting of a constitutive expression promoter, an inducible promoter and a neuron-specific expression promoter. In a more particular embodiment, the gene construct according to the invention comprises the cytomegalovirus intermediate-early (CMV IE) promoter, the sequence coding for s-KL (cDNA of mouse or human s-KL) and a polyadenylation chain (poly A). In another particular embodiment, the gene construct according to the invention comprises the CAG promoter, the sequence coding for s-KL (cDNA of mouse or human s-KL) and a polyadenylation chain (poly A).

In a particular embodiment of the third aspect, optionally in combination with any of the embodiments provided above and below, the gene construct consists in either SEQ ID NO: 7 or SEQ ID NO: 8.

All these gene constructs are able to express the protein of interest once in the cell.

In order to facilitate administration of the constructs, the invention also provides, in a fourth aspect, an expression vector comprising the gene construct as defined in the third aspect and thus comprising the nucleic acid sequence of the second aspect coding for the polypeptide of the first aspect operatively linked to an expression promoter, and particularly to a constitutive expression promoter, for use in the prevention and/or treatment of a bone disorder.

This aspect can also be formulated as the expression vector as defined above for use in the prevention and/or treatment of a bone disorder. The present invention also relates to the expression vector as defined above for use in a method for the treatment and/or prevention of a bone disorder, comprising administering a therapeutically effective amount of the expression vector as defined above, together with pharmaceutically acceptable excipients or carriers, in a subject in need thereof, including a human.

In a particular embodiment of the fourth aspect, optionally in combination with any of the embodiments provided above and below, the expression vector is a viral vector.

In a particular embodiment of the fourth aspect, optionally in combination with any of the embodiments provided above and below, the viral vector is an adeno-associated virus. In a particular embodiment, it an adeno-associated virus of serotype selected from the group consisting of AAV1, AAV2, AAV4, AAV5, AAV6, AAV7, AAV8, AAV9, AAVrh10, PHPeB, and 9P31. In a more particular embodiment, it an adeno-associated virus of serotype AAV9.

In another embodiment of the first, second, third and fourth aspects, optionally in combination with any of the embodiments provided above and below, the polypeptide for use according to the first aspect, the nucleic acid sequence for use according to the second aspect, the gene construct for use according to the third aspect, or the expression vector for use according to the fourth aspect, is administered in the form of a pharmaceutical composition together with at least one pharmaceutically acceptable excipient, diluent or carrier.

The expression "pharmaceutical composition" encompasses both compositions intended for human as well as for non-human animals. The skilled in the art understands that a pharmaceutical composition must comprise a therapeutically effective amount of the compound. The expression "therapeutically effective amount" as used herein, refers to the amount of polypeptide, nucleic acid sequence, gene construct, or expression vector that, when administered, is sufficient to prevent development of, or alleviate to some extent, one or more of the symptoms of the disease which is addressed. The particular dose of compound administered according to this invention will of course be determined by the particular circumstances surrounding the case, including the compound administered, the route of administration, the particular condition being treated, and the similar considerations.

The expression "pharmaceutically acceptable excipient, diluent or carrier" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and non-human animals without excessive toxicity, irritation, allergic response, immunogenicity or other problems or complications commensurate with a reasonable benefit/risk ratio.

Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The relative amounts of the active ingredient, the pharmaceutically acceptable excipient, and/or any additional ingredients in a pharmaceutical composition of the invention will vary, depending upon the identity, size, and/or condition of the subject treated and further depending upon the route by which the composition is to be administered.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Excipients such as coloring agents, coating agents, sweetening, and flavoring agents can be present in the composition, according to the judgment of the formulator.

The pharmaceutical compositions containing the protein or nucleic acid of the invention can be presented in any dosage form, for example, solid or liquid, and can be administered by any suitable route, for example, oral, parenteral, rectal, topical, intranasal, intraocular, intraperitoneal or sublingual route, for which they will include the pharmaceutically acceptable excipients necessary for the formulation of the desired dosage form, for example, topical formulations (ointment, creams, lipogel, hydrogel, etc.), eye drops, aerosol sprays, injectable hydrogels, injectable solutions, osmotic pumps, etc.

Exemplary diluents include, but are not limited to, calcium carbonate, sodium carbonate, calcium phosphate, dicalcium phosphate, calcium sulfate, calcium hydrogen phosphate, sodium phosphate lactose, sucrose, cellulose, microcrystalline cellulose, kaolin, mannitol, sorbitol, inositol, sodium chloride, dry starch, corn-starch, powdered sugar, and combinations thereof.

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked polyvinylpyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulfate, quaternary ammonium compounds, and combinations thereof.

Exemplary binding agents include, but are not limited to, starch (e.g., corn-starch and starch paste); gelatin; sugars (e.g., sucrose, glucose, dextrose, dextrin, molasses, lactose, lactitol, mannitol); natural and synthetic gums (e.g., acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, polyvinylpyrrolidone), magnesium aluminium silicate (Veegum), and larch arabogalactan); alginates; polyethylene oxide; polyethylene glycol; inorganic calcium salts; silicic acid; polymethacrylates; waxes; water; alcohol; and combinations thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, ascorbyl palmitate, ascorbyl stearate, ascorbyl oleate, butylated hydroxyanisole, butylated hydroxytoluene, monothioglycerol, potassium metabisulfite, propionic acid, propyl gallate, sodium ascorbate, sodium bisulfite, sodium metabisulfite, and sodium sulfite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminum hydroxide, alginic acid, pyrogen-free water, isotonic saline, Ringer's solution, ethyl alcohol, and combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulfate, sodium lauryl sulfate, and combinations thereof.

In another embodiment of the first, second, third and fourth aspects, optionally in combination with any of the embodiments provided above and below, the pharmaceutical composition is for being administered to the patient via mucosa (e.g., nasal, sublingual, vaginal, buccal, or rectal), parenterally (e.g., subcutaneous, intravenous, intramuscular, or intraarterial injection, either bolus or infusion), orally, transdermally or via inhalation by means e.g. of an aerosol. Formulations suitable for parenteral administration, such as, for example, by intraarticular, intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. Injection solutions and suspensions can also be prepared from sterile powders, granules, and tablets. In some embodiments, the composition is administered by injection e.g subcutaneous, intraperitoneal, intravesically, intravenous, intracerebroventricular, by infusion, e.g., using a reservoir or osmotic minipump or intramuscular. The formulation can be provided in unit-dose or multi-dose sealed containers, such as ampoules and vials. In an even more particular embodiment, the pharmaceutical composition is for intraventricular administration or for intravenous administration; even more particularly systemic intravenous administration.

In another embodiment of the first, second, third and fourth aspects, optionally in combination with any of the embodiments provided above and below, the polypeptide for use according to the first aspect, the nucleic acid sequence for use according to the second aspect, the gene construct for use according to the third aspect, or the expression vector for use according to the fourth aspect, is administered in combination with another active agent. Suitable active agents to be administered in combination with a compound of the invention are, without limitation, Abaloparatide, Alendronate, Ibandronate, Risedronate, Zoledronic Acid, Calcitonin, Denosumab, Bazodoxifene, Romosozumab, Abaloparatide, Estrogen, Alfacalcidol, Strontium ranelate, Eptotermin alfa, Dibotermin alfa, Ipriflavone, Aluminum chlorohydrate, Romosozumab, Burosumab, Calcium, Tiludronic acid, Etidronic acid, Cholecalciferol, Teriparatide, Abaloparatide, Vitamin D, Raloxifene, and mixtures thereof.

The embodiments of the first aspect are also meant to apply to the second, third and fourth aspects of the invention.

The inventors have thus realized that the musculoskeletal system of the animals that received a polypeptide of sequence SEQ ID NO: 1 or a variant thereof consisting of a sequence at least 85% identical to SEQ ID NO: 1, was reinforced in relation to those animals not receiving it. Thus, the polypeptides are also proposed for use in the prevention and/or treatment of a musculoskeletal disorder.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim.

### Examples

### Materials and methods

### Animal housing

SAMR1 (n=9) and SAMP8 (n=29) male mice (7-month-old) were used to perform behavioral and molecular analyses. The animals were divided randomly into three groups: SAMR1 Control (SR1 Null) (n=9), SAMP8 Control (SP8 Null) (n=9) both injected with AAV9 null, SAMP8 with injected AAV9 s-KL (SP8 s-KL) (n=11) and SAMP8 with injected AAV9 m-KL (SP8 m-KL) (n=9). For the young mice experiment, 32 C57BL/6J wild type mice (16 males and 16 females) were randomly divided into three groups: C57BL Control (n=12), C57BL s-KL (n=10) and C57BL p-KL (n=10).

Animals had free access to food and water and were kept under standard temperature conditions (22±2°C) and a 12-h light/dark cycle (300 lux/0 lux). AAV vectors were administered by intracerebroventricular injection in 7-month-old mice. Animals were deeply anesthetized by intraperitoneal injection of 10 mg/kg of ketamine (Imalgene 500, Rhone-Merieux) and 1 mg/kg of xylazine (Rompun, Bayer) diluted in NaCl 0.9%. Stereotaxic injections were performed at coordinates, -0.2 mm Antero-posterior, -2 mm Dorso-ventral, and +1 mm Medio-lateral from bregma. The vector dose was 1x10¹¹ viral genomes per animal in 6 µL, administered at a speed of 0,5µL/min using an ultramicropump (WorldPrecision Instruments). An additional AAVs dose was administered for the experiment with young mice. Young animals were administered intra tail vein injection with 4x10¹¹ viral genomes of vector, final volume of 200 µL.

Adeno-Associated Viruses (AAV) serotype 9 were generated in HEK293 cells by the triple transfection method at the Unitat de Producció de Vectors (UPV) at Universitat Autònoma de Barcelona following the protocol described previously (Piedra, J. et al., "Development of a rapid, robust, and universal picogreen-based method to titer adeno-associated vectors", Human Gene Therapy Methods, vol. 26(1), pp. 35-42). Briefly, viral vectors were precipitated using a hypersaline solution and treated with Benzonase (Novagen), followed by purification with an iodixanol density gradient (Axis-Shield PoC AS) separated by ultracentrifugation. Vector concentration was quantified using the Picogreen method (Invitrogen).

The vectors used were standard vectors containing a murine s-KL expression cassette under the control of a CAG promoter (SEQ ID NO: 7) (for aged mice) for expressing murine s-KL protein (SEQ ID NO: 2), a murine s-KL expression cassette under the control of a CMV promoter (SEQ ID NO: 8) (for young mice) for expressing murine s-KL protein (SEQ ID NO: 2); and for comparative purposes, a murine m-KL expression cassette under the control of a CMV promoter (SEQ ID NO: 9) for expressing murine m-KL (SEQ ID NO: 11), and a null expression cassette under the control of a CMV promoter (SEQ ID NO: 10) that does not express any protein.

### RNA extraction and gene expression analysis

Total RNA isolation was carried out using TRIsure^{™} reagent following the manufacturer's instructions (Bioline Reagent). The samples were homogenized using TissueLyser LT sample disruption apparatus (QIAGEN). RNA quantity and purity were measured with NanoDrop^{™} 1000 Spectrophotometer (Thermo Scientific). RNA retrotranscription was done using iScript^{™} Advanced cDNA Synthesis Kit (Bio-rad). Gene expression was analyzed by Real-Time quantitative PCR (RT-qPCR) on a Bio-Rad CFX-384 PCR machine at the Analysis and Photodocumentation Service of the Universitat Autonòma de Barcelona. Each reaction contained 25 ng of cDNA, 7.5 µL of iTaqTM Universal SYBR Green Supermix (Bio-Rad) and a primer concentration of 0.2 nM, with a reaction final volume of 15 µL. Primers used were the following:

| Target Gene | Reference or sequence |
|---|---|
| *Acp5 (TRAP)* | Mm00475698_m1 (ThermoFisher) |
| *Bglap (Osteocalcin)* | Mm03413826_mH (ThermoFisher) |
| *Bmp2* | Mm01340178_m1 (ThermoFisher) |
| *c-Fos* | Mm00487425_m1 (ThermoFisher) |
| *Col1a1* | Mm00801666_g1 (ThermoFisher) |
| *Ctsk (Cathepsin K)* | Mm00484039_m1 (ThermoFisher) |
| *Dmp1* | Mm01208363_m1 (ThermoFisher) |
| *Egr1* | Mm00656724_m1 (ThermoFisher) |
| *Fgf23* | Mm00445621_m1 (ThermoFisher) |
| *Gapdh* | Mm 99999915_g1 (ThermoFisher) |
| *NCC* | Mm01173990_m1 (ThermoFisher) |
| *Npt2c* | Mm00551746_m1 (ThermoFisher) |
| *Runx2* | Mm03003491_m1 (ThermoFisher) |
| *Romk* | Mm01173990_m1 (ThermoFisher) |
| *Spp1 (Osteopontin)* | Mm00436767_m1 (ThermoFisher) |
| *Sp7 (Osterix)* | Mm00504574_m1 (ThermoFisher) |
| *Tbp* | Mm00446973_m1 (ThermoFisher) |
| *Tnfsf11 (RANKL)* | Mm00441906_m1 (ThermoFisher) |
| *Tnfrsf11b (Opg)* | Mm00435454_m1 (ThermoFisher) |
| *Trpv5* | Mm01166037_m1 (ThermoFisher) |
| *Cyp24a1* | Fw: CTGCCCCATTGACAAAAGGC (SEQ ID NO: 12) |
| | Rv: CTCACCGTCGGTCATCAGC(SEQ ID NO: 13) |
| *Cyp27b1* | Fw: GCACAGTTTACGTTGCCGAC (SEQ ID NO: 14) |
| | Rv: CGTTAGCAATCCGCAAGCA (SEQ ID NO: 15) |

The analysis of qPCR data was done following the ΔΔCt method. Cycle threshold (Ct) were normalized subtracting to each experimental Ct, the difference of its housekeeping (HK) Ct respect the HK's average value. HK genes used were β-actin and GAPDH. Melting curves were also analyzed to ensure unique amplificon generation. Each sample (n=6 per group) was tested at least in duplicates, and Cts higher than 37 cycles were considered as no amplified.

### Biochemical study

Blood extraction was done by the tail snip procedure using microvette tubes (Sarstedt, Germany). Tubes were centrifuged 5 min at 10000 g and serum was isolated and preserved at -80°C. Ion concentration in serum was measured using the Olympus AU480 analyzer (Germany) following the arsenazo III method for calcium and phosphomolybdate (340 nm) for phosphate determination.

### Bone study

Right legs of perfused animals were isolated and placed in a PFA 4% solution for preservation. Tibias were dissected, eliminating muscle tissue, and placed in PBS solution with 0,05% sodium azide (NaN3). MicroCT analysis was done with a SkyScan 1272 (Bruker) computerized microtomography imaging system at the Centre de Recerca en Ciència i Enginyeria Multiescala de Barcelona (CRCEMB) at Universitat Politècnica de Catalunya (UPC).

Images were reconstructed with the NRecon v1.6 (Bruker) program and analyzed with the CT-Analyser v1.13 image program (Bruker). Cortical bone analysis was done with 100 slices by manually selecting volumes of interest (VOIs), with a binary threshold of 50-255. Analyzed image intervals started from peronei insertion towards upper tibial epiphysis. For trabecular bone analysis, 150 slices were analyzed with a binary threshold of 20-255. All selected interval of images started 100 VOIs from the femoral distal growth plate towards tibial diaphysis. Finally, 3D representations of the bones were obtained with the CTVox v3.3 program (Bruker).

Mineral density was calculated with the CT-Analyzer v1.13 program calibrating bone absorbance with two 2mm diameter hydroxyapatite phantoms (Bruker-MicroCT) of known density of 0.25 and 0.75 g/cm3.

### Bone fracture test

The whole isolated bone was placed in a fixture attached to a material testing machine and loaded until broken. At the start of the three-point bending test, the bone was in undeformed state (zero displacement), and the loading point made contact with the bone with a small pre-load (< 1 N) in order to keep the bone in place. The loading point then moved progressively (1 µm/s) downward with increasing applied load (or force, F) and displacement (d) until complete failure. Load and displacement values were recorded during the test and stored in a data file.

### Statistical analysis

Statistical analysis and graphic representation were done with GraphPad Prism ver.6 (GraphPad Software). Statistical differences between groups were analyzed with a two-tailed unpaired Student's t-test when comparing two groups, and One-Way analysis of variance (ANOVA), followed by Tukey as a post-hoc analysis when it was necessary. Data are expressed as mean ± Standard Error of the Mean (SEM). Statistical difference was accepted when p values were ≤ 0,05 and outliers were detected by Grubb's test and removed from the analysis.

### Results

### Effect of KL isoforms over young WT animals' mineral metabolism

Serum levels of ions related with bone mineral homeostasis were altered one month after AAV treatment expressing p-KL (Fig. 1) of young mice. A significant reduction of both ions (phosphate and calcium) was detected in C57BL/6J mice treated with p-KL compared with both Null and s-KL treated animals.

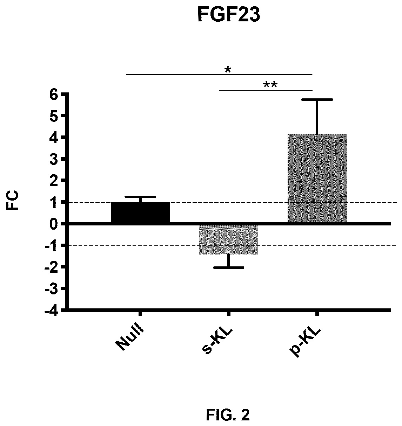

FGF23 is a hormone expressed in osteocytes of the bone tissue and it participates in ion regulation by decreasing phosphate levels and promoting vitamin D degradation. In accordance with the biochemical results, expression of p-KL isoform in young animals increased 4 times *fgf23* gene expression in bones (Fig. 2). In contrast, s-KL isoform did not alter *fgf23* expression, presenting mRNA levels comparable to the Null group.

MicroCT analysis of tibia structure two months after treatment revealed strong alterations in some cortical and trabecular parameters following the p-KL treatment but not with s-KL or Null treated animals (Fig. 3). A 10% reduction in cortical bone volume and area was observed after p-KL treatment. Cortical bone thickness (Cs.Th) was also significant reduced just with the p-KL isoform. Trabecular bone presented a 47% reduction in bone volume after p-KL treatment, due to a significant reduction in trabecular number (44% trabecular loss). Surprisingly, bones of animals treated with the s-KL vector presented a tendency to increase cortical and trabecular bone volume, presenting a significant increase in trabecular thickness when compared to null animals.

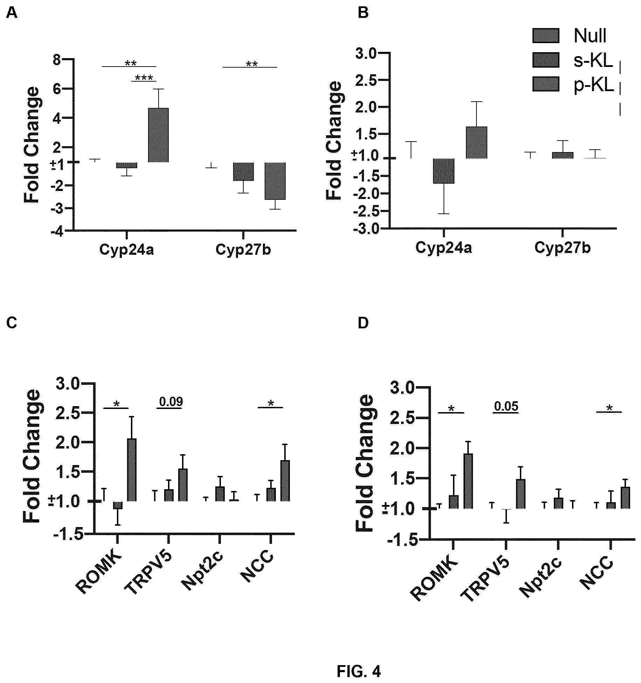

As shown in Fig. 4 the long-term expression of p-KL significantly deregulated Vitamin D metabolism in males and also, significantly deregulated important ion channels in kidney, while s-KL did not induce any of these deleterious effects.

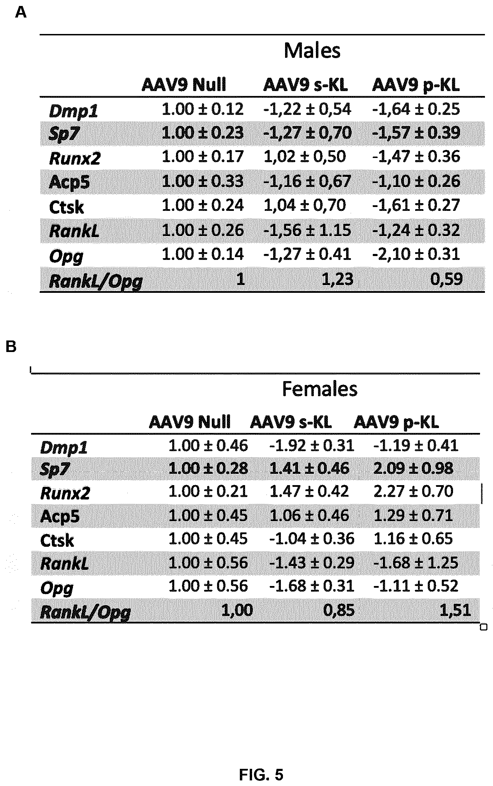

A shown in Fig.5, the long-term expression of p-KL significantly deregulated the expression of FGF23 and bone specific genes, both in males and females but s-KL did not show such negative effects.

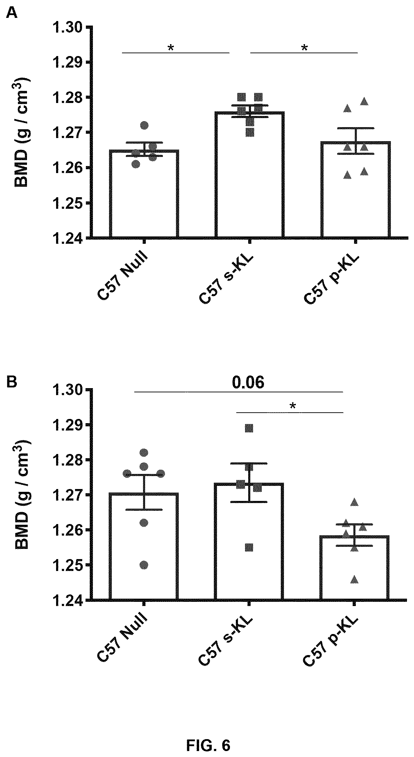

Fig. 6 shows that the long-term expression of p-KL significantly reduced bone mineral density especially in females, while in contrast, s-KL expression did not reduce, but instead, significantly increased bone mineral density in males.

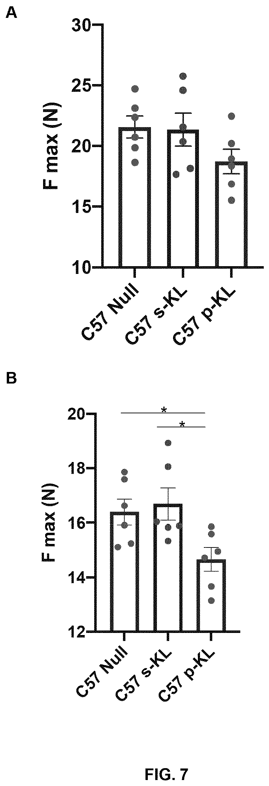

As shown in Fig. 7, the long-term expression of p-KL significantly increased bone fragility and reduced resistance to fracture (both in males and females), while s-KL did not show any of these deleterious effects.

### AAV administration efficiently expressed KL in SAMP8 animals

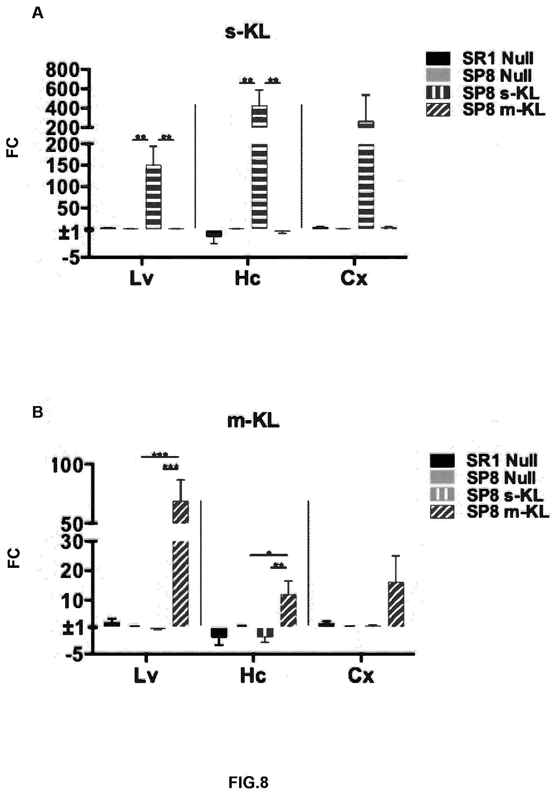

To efficiently transduce neuronal and liver cell types, the AAV serotype 9 (AAV9) as gene therapy vector was chosen. Three different AAV9 vectors were generated containing expression vectors for producing the secreted (SEQ ID NO: 7) and the transmembrane (SEQ ID NO: 9) Klotho isoforms as well as a Null control vector (SEQ ID NO: 10). Intraventricular administration was done in 7-month-old mice and a therapeutic period of 10 weeks was set, finishing the experiment when animals were 10-month-old as previously described (Massó A. et al., " Secreted αKlotho isoform protects against age-dependent memory deficits" Mol Psychiatry, 2018, vol. 23(9), pp. 1937-1947). The effectiveness of AAV9-mediated expression of m-KL and s-KL was evaluated by RNA analysis from different tissues. As seen in Fig. 8, both AAV9 s-KL and m-KL mediated strong αKlotho expression after AAV injection in the different areas analyzed. Vector expression was associated with an increase in KL protein in the hippocampus, area of special interest due to its importance in learning and memory formation.

### KL treatment reverses structural changes observed in aged SAMP8 tibia

Effects of KL treatment over the microstructure of long bone tibia are shown in Table 1:

**Table 1**

| | **SR1 Null** | **SP8 Null** | **SP8 s-KL** | **SP8 m-KL** |
|---|---|---|---|---|
| Periosteal perimeter (mm) | 6.34 ± 0.24 ^{a} | 6.65 ± 0.10 | 6.11 ± 0.40 ^{b} | 6.35 ± 0.20 |
| Cortical area (mm²) | 0.75 ± 0.05 ^{a} | 0.87 ± 0.08 | 0.75 ± 0.06 ^{b} | 0.76 ± 0.06 ^{b} |
| Cortical thickness | 0.24 ± 0.01 | 0.25 ± 0.01 | 0.25 ± 0.01 | 0.24 ± 0.02 |
| Endocortical perimeter (mm) | 2.15 ± 0.10 ^{a} | 2.28 ± 0.05 | 2.00 ± 0.16 ^{b} | 2.18 ± 0.11 ^{c} |
| Medullary area (mm²) | 0.30 ± 0.03 ^{a} | 0.35 ± 0.02 | 0.27 ± 0.04 ^{b} | 0.32 ± 0.03 |
| Cortical BMD | 1.22 ± 0.07 | 1.23 ± 0.07 | 1.21 ± 0.10 | 1.18 ± 0.05 |
| Tibial length (mm) | 16.14 ± 0.23 | 16.24 ± 0.13 | 16.17 ± 0.20 | 16.26 ± 0.08 |

Data expressed as mean ± SD. ^{a} Statistical differences p< 0.05 between SAMR1 and SAMP8 Null.^{b} Statistical differences p< 0.05 between SAMP8 Null and treated SAMP8.

Mid-tibial cortical bone presented a diameter expansion in SAMP8 Null mice (Table 1). This corresponds to a 5% perimeter and 16% cortical area expansion of SAMP8 compared to SAMR1 cortical area. SAMP8 medullary space was also increased 6% and 17% perimeter and endocortical area respectively. Of note, Klotho treatment significantly prevented cortical expansion, with parameters equivalent to SAMR1 animals (Table 1). Interestingly, a higher reduction in periosteal and endocortical perimeters was detected with s-KL treatment compared to m-KL. In addition, other important parameters for determining bone strength and elasticity such as bone length, cortical thickness or bone mineral density were not altered between SAMP8 and SAMR1 mice, or after the treatment. Percentage of trabecular bone volume (BV/TV) was reduced in SAMP8 model compared to SAMR1, which is compatible with the bone loss associated to the strain, a feature not affected by the KL treatment (Table 1).

The effect of secreted KL treatment was only detectable in SAMP8 mice, as SAMR1 animals treated with s-KL did not show alterations in the analyzed cortical or trabecular parameters compared to control SR1 animals. Relevance in bone strength of the detected structural alteration was assessed with a 3-points bending fracture test. A tendency was observed to increased bone plasticity under stress with KL treatments.

### Citation List

Minamizaki T. et al., "Soluble Klotho causes hypomineralization in Klotho-deficient mice" J Endocrinol., 2018, vol. 237(3), pp. 285-300.
Maruyama, N., et al., "Bone micro-fragility caused by the mimetic aging processes in alpha-klotho deficient mice: in situ nanoindentation assessment of dilatational bands", Biomaterials, 2015. Vol. 47, pp. 62-71.
Kawaguchi, H., et al., "Independent impairment of osteoblast and osteoclast differentiation in klotho mouse exhibiting low-turnover osteopenia" J Clin Invest, 1999, vol. 104(3), p. 229-37.
Suzuki, H., et al., "Histological and elemental analyses of impaired bone mineralization in klotho-deficient mice" J Anat, 2008. vol. 212(3), pp. 275-85.
Matsumura et al., "Identification of the human klotho gene and its two transcripts encoding membrane and secreted Klotho protein", Biochem Biophys Res Commun-1998, vol. No. 242, pp.:626-630
Altschul et al., "Basic local alignment search tool", 1990, J. Mol. Biol, vol. 215, pp. 403-410.
Massó A. et al., " Secreted αKlotho isoform protects against age-dependent memory deficits" Mol Psychiatry, 2018, vol. 23(9), pp. 1937-1947
Piedra, J. et al., "Development of a rapid, robust, and universal picogreen-based method to titer adeno-associated vectors", Human Gene Therapy Methods, vol. 26(1), pp. 35-42

## Claims

1. Polypeptide consisting of sequence SEQ ID NO: 1, or a variant thereof consisting of a sequence at least 85% identical to SEQ ID NO: 1, for use in the prevention and/or treatment of a bone disorder.

2. The polypeptide for use according to claim 1, wherein bone disorder is bone degeneration and/or bone loss.

3. The polypeptide for use according to any of claims 1-2, wherein the bone disorder is age-related bone degeneration and/or bone loss.

4. The polypeptide for use according to any of claims 1-3, wherein the bone disorder is osteopenia and/or osteoporosis.

5. The polypeptide for use according to any of claims 1-4, wherein the polypeptide consists of sequence SEQ ID NO: 1 or a variant thereof consisting of a sequence at least 88 % identical to SEQ ID NO: 1.

6. The polypeptide for use according to any of claims 1-5, wherein the polypeptide consists of sequence SEQ ID NO: 1 or a variant thereof consisting of a sequence at least 98 % identical to SEQ ID NO: 1.

7. The polypeptide for use according to any of claims 1-5, wherein the polypeptide consists of SEQ ID NO: 1 or SEQ ID NO: 2.

8. Nucleic acid sequence that encodes the polypeptide or the variant thereof as defined in any of claims 1-7, for use in the prevention and/or treatment of a bone disorder.

9. Gene construct comprising a nucleic acid sequence as defined in claim 8, operatively linked to an expression promoter, for use in the prevention and/or treatment of a bone disorder.

10. Expression vector comprising the gene construct as defined in claim 9, for use in the prevention and/or treatment of a bone disorder.

11. The expression vector for use according to claim 10, which is a viral vector.

12. The expression vector for use according to claim 11, which is an adeno-associated virus of serotype selected from the group consisting of AAV1, AAV2, AAV4, AAV5, AAV6, AAV7, AAV8, AAV9, AAVrh10, PHPeB, and 9P31.

13. The polypeptide for use according to any of claims 1-7, the nucleic acid sequence for use according to claim 8, the gene construct for use according to claim 9, or the expression vector for use according to any of claims 10-12, which is administered in the form of a pharmaceutical composition together with at least one pharmaceutically acceptable excipient, diluent or carrier.

14. The polypeptide, the nucleic acid sequence, the gene construct, or the expression vector for use according to claim 13, wherein the pharmaceutical composition is for intraventricular administration or for intravenous administration.

15. The polypeptide for use according to any of claims 1-7, the nucleic acid sequence for use according to claim 8, the gene construct for use according to claim 9, or the expression vector for use according to any of claims 10-12, which is administered in combination with another active agent.

## Patentansprüche

1. Polypeptid bestehend aus der Sequenz SEQ ID NO: 1, oder einer Variante davon, die aus einer Sequenz besteht, die zu mindestens 85 % identisch mit der SEQ ID NO: 1 ist, zur Verwendung bei der Prävention und/oder Behandlung einer Knochenerkrankung.

2. Das Polypeptid zur Verwendung nach Anspruch 1, wobei die Knochenerkrankung Knochendegeneration und/oder Knochenverlust ist.

3. Das Polypeptid zur Verwendung nach einem der Ansprüche 1 bis 2, wobei die Knochenerkrankung eine altersbedingte Knochendegeneration und/oder ein altersbedingter Knochenverlust ist.

4. Das Polypeptid zur Verwendung nach einem der Ansprüche 1 bis 3, wobei die Knochenerkrankung Osteopenie und/oder Osteoporose ist.

5. Das Polypeptid zur Verwendung nach einem der Ansprüche 1 bis 4, wobei das Polypeptid aus der Sequenz SEQ ID NO: 1, oder einer Variante davon besteht, die aus einer Sequenz besteht, die zu mindestens 88 % identisch ist mit der SEQ ID NO: 1.

6. Das Polypeptid zur Verwendung nach einem der Ansprüche 1 bis 5, wobei das Polypeptid aus der Sequenz SEQ ID NO: 1 oder einer Variante davon besteht, die aus einer Sequenz besteht, die zu mindestens 98 % identisch ist mit der SEQ ID NO: 1.

7. Das Polypeptid zur Verwendung nach einem der Ansprüche 1 bis 5, wobei das Polypeptid aus der SEQ ID NO: 1 oder der SEQ ID NO: 2 besteht.

8. Nukleinsäuresequenz, die das Polypeptid oder die Variante davon wie in einem der Ansprüche 1 bis 7 definiert kodiert, zur Verwendung bei der Prävention und/oder Behandlung einer Knochenerkrankung.

9. Genkonstrukt, umfassend eine Nukleinsäuresequenz wie in Anspruch 8 definiert, operativ verbunden mit einem Expressionspromotor, zur Verwendung bei der Prävention und/oder Behandlung einer Knochenerkrankung.

10. Expressionsvektor, umfassend das Genkonstrukt wie in Anspruch 9 definiert, zur Verwendung bei der Prävention und/oder Behandlung einer Knochenerkrankung.

11. Der Expressionsvektor zur Verwendung nach Anspruch 10, welcher ein viraler Vektor ist.

12. Der Expressionsvektor zur Verwendung nach Anspruch 11, welcher ein adenoassoziiertes Virus eines Serotyps ausgewählt aus der Gruppe bestehend aus AAV1, AAV2, AAV4, AAV5, AAV6, AAV7, AAV8, AAV9, AAVrh10, PHPeB und 9P31 ist.

13. Das Polypeptid zur Verwendung nach einem der Ansprüche 1 bis 7, die Nukleinsäuresequenz zur Verwendung nach Anspruch 8, das Genkonstrukt zur Verwendung nach Anspruch 9 oder der Expressionsvektor zur Verwendung nach einem der Ansprüche 10 bis 12, die/das/der in Form einer pharmazeutischen Zusammensetzung zusammen mit mindestens einem pharmazeutisch akzeptablen Hilfsstoff, Verdünnungsmittel oder Träger verabreicht wird.

14. Das Polypeptid, die Nukleinsäuresequenz, das Genkonstrukt oder der Expressionsvektor zur Verwendung nach Anspruch 13, wobei die pharmazeutische Zusammensetzung zur intraventrikulären Verabreichung oder zur intravenösen Verabreichung bestimmt ist.

15. Das Polypeptid zur Verwendung nach einem der Ansprüche 1 bis 7, die Nukleinsäuresequenz zur Verwendung nach Anspruch 8, das Genkonstrukt zur Verwendung nach Anspruch 9 oder der Expressionsvektor zur Verwendung nach einem der Ansprüche 10 bis 12, das/die/der in Kombination mit einem anderen Wirkstoff verabreicht wird.

## Revendications

1. Polypeptide constitué de la séquence SEQ ID n ° : 1, ou une variante de celle-ci consistant en une séquence au moins 85 % identique à la SEQ ID n ° : 1, pour l'utilisation dans la prévention et/ou le traitement d'un trouble osseux.

2. Le polypeptide pour l'utilisation selon la revendication 1, dans lequel le trouble osseux est la dégénérescence osseuse et/ou la perte osseuse.

3. Le polypeptide pour l'utilisation selon l'une quelconque des revendications 1 à 2, dans lequel le trouble osseux est la dégénérescence osseuse et/ou la perte osseuse liée(s) à l'âge.

4. Le polypeptide pour l'utilisation selon l'une quelconque des revendications 1 à 3, dans lequel le trouble osseux est l'ostéopénie et/ou l'ostéoporose.

5. Le polypeptide pour l'utilisation selon l'une quelconque des revendications 1 à 4, dans lequel le polypeptide consiste en la séquence SEQ ID n ° : 1, ou une variante de celle-ci consistant en une séquence au moins 88 % identique à la SEQ ID n ° : 1.

6. Le polypeptide pour l'utilisation selon l'une quelconque des revendications 1 à 5, dans lequel le polypeptide consiste en la séquence SEQ ID n ° : 1, ou une variante de celle-ci consistant en une séquence au moins 98 % identique à la SEQ ID n ° : 1.

7. Le polypeptide pour l'utilisation selon l'une quelconque des revendications 1 à 5, dans lequel le polypeptide consiste en la SEQ ID n ° : 1 ou bien la SEQ ID n °: 2.

8. Séquence d'acide nucléique qui code pour le polypeptide ou son variant tel que défini dans l'une quelconque des revendications 1 à 7, pour l'utilisation dans la prévention et/ou le traitement d'un trouble osseux.

9. Construction génique comprenant une séquence d'acide nucléique telle que définie dans la revendication 8, liée de manière opérationnelle à un promoteur d'expression, pour l'utilisation dans la prévention et/ou le traitement d'un trouble osseux.

10. Vecteur d'expression comprenant la construction génique telle que définie dans la revendication 9, pour l'utilisation dans la prévention et/ou le traitement d'un trouble osseux.

11. Le vecteur d'expression pour l'utilisation selon la revendication 10, qui est un vecteur viral.

12. Le vecteur d'expression pour l'utilisation selon la revendication 11, qui est un virus adéno-associé de sérotype choisi dans le groupe constitué par AAV1, AAV2, AAV4, AAV5, AAV6, AAV7, AAV8, AAV9, AAVrh10, PHPeB et 9P31.

13. Le polypeptide pour l'utilisation selon l'une quelconque des revendications 1 à 7, la séquence d'acide nucléique pour l'utilisation selon la revendication 8, la construction génique pour l'utilisation selon la revendication 9, ou le vecteur d'expression pour l'utilisation selon l'une quelconque des revendications 10 à 12, qui est administré(e) sous la forme d'une composition pharmaceutique avec au moins un excipient, diluant ou véhicule pharmaceutiquement acceptable.

14. Le polypeptide, la séquence d'acide nucléique, la construction génique ou le vecteur d'expression pour l'utilisation selon la revendication 13, dans lequel la composition pharmaceutique est pour l'administration intraventriculaire ou pour l'administration intraveineuse.

15. Le polypeptide pour l'utilisation selon l'une quelconque des revendications 1 à 7, la séquence d'acide nucléique pour l'utilisation selon la revendication 8, la construction génique pour l'utilisation selon la revendication 9, ou le vecteur d'expression pour l'utilisation selon l'une quelconque des revendications 10 à 12, qui est administré(e) en combinaison avec un autre agent actif.
